# EUROPEAN PATENT SPECIFICATION

(11) **EP 2 109 589 B1**
(45) Date of publication and mention of the grant of the patent: **02.04.2014**
(21) Application number: 07859187.2
(22) Date of filing: 27.12.2007
(51) Int. Cl.: C02F 3/28

(54) **PRODUCTION OF HYDROGEN AND METHANE FROM WASTEWATER STREAMS**
HERSTELLUNG VON WASSERSTOFF UND METHAN AUS ABWASSERSTRÖMEN
PRODUCTION D'HYDROGÈNE ET DE MÉTHANE À PARTIR DE COURANTS D'EAUX USÉES

(30) Priority: 28.12.2006 ZA 200605326
(43) Date of publication of application: 21.10.2009
(73) Proprietor: University Of The Witwatersrand, Johannesburg, Braamfontein Johannesburg (ZA)
(72) Inventor: GRAY, Vincent Myles, Braamfontein, Johannesburg (ZA)
(74) Representative: Cremer & Cremer
(86) International application number: PCT/IB2007/004100
(87) International publication number: WO 2008/081292

(56) References cited:
- GB-A- 2 054 549
- US-A- 4 845 034
- US-A- 5 076 927
- US-A- 5 207 911
- US-A1- 2005 252 858
- PEREZ M ET AL: "Comparative performance of high rate anaerobic thermophilic technologies treating industrial wastewater" WATER RESEARCH, ELSEVIER, AMSTERDAM, NL, vol. 32, no. 3, 1 March 1998 (1998-03-01), pages 559-564, XP004107524 ISSN: 0043-1354
- CHARLES S RABKIN ET AL: 'Thermophilic bacteria: a New cause of Human Disease' JOURNAL OF CLINICAL MICROBIOLOGY, [Online] 01 April 1985, pages 553 - 557, XP055073939 Retrieved from the Internet: <URL:http://jcm.asm.org/content/21/4/553.fu ll.pdf> [retrieved on 2013-08-02]
- Metcalf & Eddy: "Wastewater Engineering, Treatment and Reuse, 4th edition", 2003, Mc Graw Hill, Boston ISBN: 0-07-041878-0 pages 1529-1530,
- OTAJEVWO ET AL: 'Cultural Conditions Necessary for Optimal Cellulase Yield by Cellulolytic Bacterial Organisms as They Relate to Residual Sugars Released in Broth Medium' MODERN APPLIED SCIENCE vol. 5, no. 3, 02 June 2011, XP055073950 DOI: 10.5539/mas.v5n3p141 ISSN: 1913-1844

## Description

### FIELD OF THE INVENTION

This invention relates to production of hydrogen and methane from wastewater streams and, more particularly, to the treatment of wastewater so as to facilitate the production of hydrogen and methane in a fluidised bed biofilm reactor.

### BACKGROUND TO THE INVENTION

Raw or untreated sewage wastewater contains many different species of pathogenic organisms which are harmful to humans, particularly in underdeveloped regions where inhabitants use national rivers as a source of drinking water and to cook and wash in. Often the water is collected downstream from an outlet of a sewage or wastewater treatment facility.

While sterilization of large volumes of sewage to inactivate pathogens can be carried out, it is expensive and strict controls are often overlooked. This exacerbates the above problem.

One method of sterilising large volumes of sewage on a continuous basis involves the direct injection of pressurized steam into the sewage. This is done by direct injection or in an infusion chamber.

By modem sewage treatment plants biogas (gas produced by micro organisms, usually anaerobic bacteria) is used to generate the steam thus reducing the sewage treatment facility's reliance on electricity from a national grid. This, however, has a disadvantage In that steam sterilization is not selective and also removes many potentially beneficial micro organisms that are normally involved in the production of the hydrogen and methane which are the main components of biogas.

Bacteria used to produce biogas are well known In the art. Typically they. are anaerobic bacteria and they may be mesophilic or thermophilic. Examples of two groups of anaerobic thermophilic bacteria are those of the genus *Thermoanaerobacter,* particularly *T. ethanolicus, T. thermohydrosulfuricus* and *T*. *brockii,* and *Clostridium,* particularly C. thermocellum and C. *thermohydrosulfuricum.*

While biogas can be produced in any one of a number of bioreactors, fluidised bed bioreactors are becoming more common. Usually fluidised bed systems have a single inlet at the base of the fluidised bed. The bed of a bioreactor is fluidised by increasing the inlet velocity of the fluid at the bottom of the bioreactor. A single inlet gives rise to a fluid flow that is parallel to the long (vertical) axis of the bioreactor at the base of bioreactor but is prone to become restricted to channels of least resistance. These channels usually form on the side of the bioreactor, very close to the walls of the bioreactor vessel.

Inefficiency in mass transfer, such as the non-dispersion of solutes uniformly throughout the bioreactor bed, is a major negative consequence of flow channeling in the bioreactor bed. This results in uneven growth and development of biofilm or microbial granules on the carrier particles in the bioreactor. Also, biofilm growth and granule formation only occur in the top section of the carrier particle bed. Biofilm and granule formation do not occur in the rest of the bed below this top section. Consequently, no biofilm or granule bed growth from the base of the bioreactor occurs, and also no bed expansion takes place.

Perez et al. (Comparative performance of high rate anaerobic thermophilic technologies treating industrial wastewater, Water Research, vol. 32, 1998, p. 559-564) disclose a one-step process wherein distillery wastewater is treated under anaerobic thermophilic conditions utilizing a fluidised bed reactor at laboratory-scale for the production of methane and hydrogen. The wastewater is heated to 55°C and maintained at this temperature for biogas production.

In US 4,845,034 anaerobic digestion of wastewater streams in subterranean cavities is disclosed. An example of an application is gasification. The process used is a one-step process.

### OBJECT OF THE INVENTION

It is an object of the invention to provide a process for the production of hydrogen and methane from wastewater streams and, thus, facilitating the production.

### SUMMARY OF THE INVENTION

In accordance with this invention there is provided a process for the production of hydrogen and methane from wastewater streams thus, at least partly, rendering said wastewater safe for use in a bioreactor process plant and rendering the wastewater suitable for the production of biological gasses, said method comprising receiving a primary untreated wastewater stream, heating said untreated wastewater stream to a first predetermined temperature for a predetermined time to, in use, inactivate pathogenic micro-organisms in the wastewater stream, allowing the heated wastewater to cool to a second predetermined temperature which, in use, facilitates growth, in the wastewater stream, of anaerobic thermophilic bacteria, maintaining the second predetermined temperature of the wastewater stream while passing said stream through a fluidised bed biofilm reactor within which are desired anaerobic thermophilic bacteria, decanting, clarifying and separating fermentation products produced in the reactor from the wastewater outflow from the biorector and returning the treated wastewater to the environment.

There is also provided for the first predetermined temperature to be approximately 95 °C, for the predetermined time at which the wastewater to be maintained at the first predetermined temperature to be between 10 and 20 minutes, for the second predetermined temperature to be not less than 70°C and preferably between 70°C and 80°C.

There is further provided for the wastewater stream to be continuously enriched, by inoculation, with appropriate anaerobic thermophilic bacteria after it has cooled to its second predetermined temperature and for the appropriate anaerobic thermophilic bacteria to have a high capacity for hydrogen and/or methane production.

There is also provided for hydrogen and/or methane produced by the anaerobic thermophilic bacteria to be used to provide at least part of the energy required to heat the untreated wastewater stream to its first predetermined temperature and/or to maintain the second predetermined temperature of the wastewater stream and for excess hydrogen and/or methane to be used in a variety of other applications such as, for example, to generate electricity.

The invention extends to a bioreactor for use in a process for the production of hydrogen and methane from wastewater streams thus, at least partly, rendering said wastewater safe for use in a bioreactor process plant and rendering the wastewater suitable for the production of biological gasses, the reactor having a primary untreated wastewater stream inlet, a heater for heating the untreated wastewater stream to a first predetermined temperature for a predetermined time to, in use, inactivate pathogenic micro-organisms in the wastewater stream, a cooling circuit within which the heated wastewater cools to a second predetermined temperature, a fluidised bed biofilm reactor containing desired anaerobic thermophilic bacteria and an outflow for treated wastewater into the environment, the wastewater in the fluidised biofilm reactor having a retention time within said reactor sufficient to maintain wastewater in the reactor substantially at the second predetermined temperature.

There is also provided for the first predetermined temperature to be approximately 95°C, for the predetermined time at which the wastewater to be maintained at the first predetermined temperature to be between 10 and 20 minutes, for the second predetermined temperature to be not less than 70°C and preferably between 70°C and 80°C.

There is further provided for the wastewater stream to be continuously enriched, by inoculation, with appropriate anaerobic thermophilic bacteria after it has cooled to its second predetermined temperature and for the appropriate anaerobic thermophilic bacteria to have a high capacity for hydrogen and/or methane production.

There is also provided for the reactor to have a wastewater heater which utilises at least some of the hydrogen and/or methane produced by the anaerobic thermophilic bacteria to provide at least part of the energy required to heat the untreated wastewater stream to its first predetermined temperature and/or to maintain the second predetermined temperature of the wastewater stream and for excess hydrogen and/or methane to be used in a variety of other applications such as, for example, to generate electricity.

The invention also extends to a fluidised bed biofilm reactor for use in the above-described process, the bioreactor having at least one vertical flow inlet and additionally one or more, preferably at least three, non-vertical or side flow inlets.

There is also provided for the side flow inlets to be oriented at between 30 and 90 degrees to the vertical axis of the fluidised bed system and for the side flow inlets, when not horizontally orientated, to be directed towards the top of the fluidised mass in the bed.

There is further provided for the openings of the side flow inlets into the fluidised bed reactor to be flush with the fluidised bed system interior surface alternatively, for the openings of the side flow inlets into the fluidised bed reactor to project beyond the interior surface of the reactor.

### BRIEF DESCRIPTION OF THE DRAWINGS

One embodiment of the invention will be described below by way of example only and with reference to the accompanying drawings in which:
- Figure 1: is a flow diagram of a process for the production of biogas;
- Figure 2: is a flow diagram of a process for the production of hydrogen and methane from a wastewater stream according to the invention; and
- Figure 3: is a schematic sectional side view of one embodiment of a fluidised bed biofilm reactor according to the invention.

### DESCRIPTION OF PREFERRED EMBODIMENT OF THE INVENTION

In the embodiment of the invention described here, wastewater from a sewage works is used to generate biogas in the form of hydrogen and methane. Sewage works represent ideal sites for the production of biogas in the form of hydrogen and methane that can be used to generate electricity to supplement the supply of the existing power supply grids. Suitable solid oxide fuel cells (SOFCs) stacks can readily generate electricity from low pressure gas streams containing hydrogen, methane and carbon monoxide. In order to make the biogas and electricity generation from sewage, a process that is not harmful to human health is required, and it would be necessary to remove all pathogenic agents by: 1) heat treatment of the primary sewage sludge and 2) maintenance of the sewage streams at temperature not below 70°C. The bioreactor process of this invention generates the energy fuels in the form of methane and microbial sludge that can be used for the low cost production of steam for both electricity generation via steam turbines and for the heating of the sewage stream.

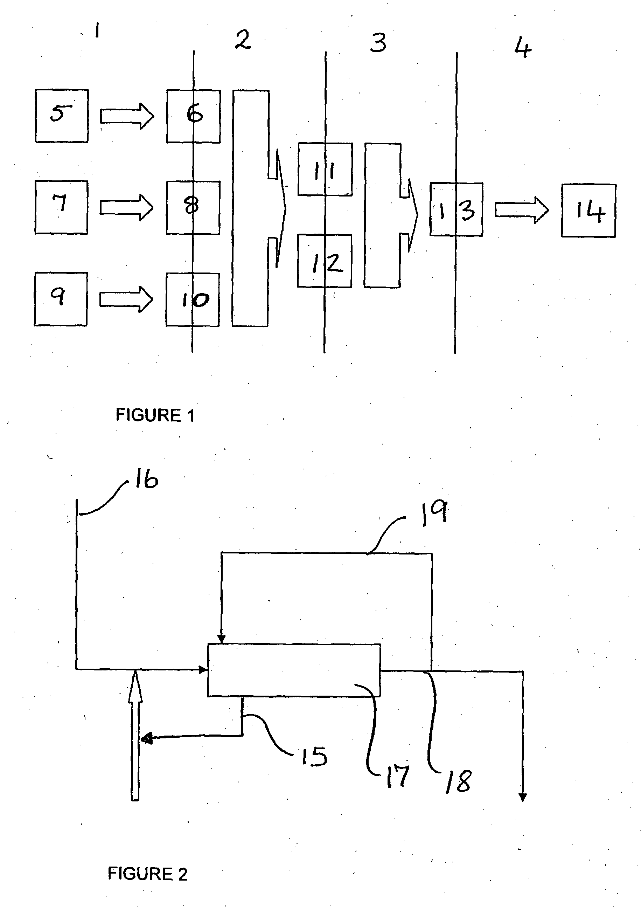

Referring now to the drawings, a general flow diagram of a process for the production of biogas is illustrated In Figure 1. The process comprises four stages namely a hydrolysis stage (1), an acidogenesis stage (2), an acetogenesis stage (3) and a methanogenesis stage (4). In the hydrolysis stage (1) carbohydrates (5) are converted into sugars (6), fats (7) are converted into fatty acids (8) and proteins (9) are converted into amino acids (10). In the acidogenesis stage (2) the sugars (6), fatty acids (8) and amino acids (10) are converted Into carbonic acids and alcohols (11) and hydrogen, carbon dioxide and ammonia (12). The carbonic acids and alcohols (11) and hydrogen, carbon dioxide and ammonia (12) are in tum converted into hydrogen, acetic acid and carbon dioxide (13) in the acetogenesis stage (3) and, In the methanogenesis stage (4) the methane and more carbon dioxide (14) are formed. The hydrogen and methane form the basis of the biogas.

Refering to Figure 2, a flow diagram of a process for the production of a biogas (15) from a wastewater stream (16) according to the invention is shown. In this embodiment the conversion of wastewater (16) to biogas takes place in a fluidised bed biofilm bioreactor (17) and it follows the path illustrated in Figure 1. In the inventive process however the wastewater stream (16) is heated to 95°C and maintained at this temperature for 10 to 20 minutes before being allowed to cool to between 70 and 80°C which is the temperature range for hydrogen and methane producing extreme thermophilic anaerobic bacterial consortia in the reactor (17). The thermophilic anaerobic bacterial consortium is also enriched by inoculating the stream flowing into the reactor (17) with an inoculum obtained from the reactor outflow (18). The inoculation is in the form of a recycling loop (19). It is envisaged that at least some of the biogas produced by the reactor will be used to provide energy to heat the wastewater stream (16).

It is envisaged that this pretreatment and operational temperature facilitates the selective enrichment of the sewage stream with extreme thermophilic anaerobic bacteria consortia. This pretreatment and operational temperatures used also remove many of the harmful pathogenic micro-organisms from the sewage stream. The heated sewage and surviving bacterial inoculum then passed through a series of fluidised bed biofilm bioreactor-decanter-clarifier-gas separator assemblies that are designed to facilitate the following processes: 1) generation of hydrogen and acetic acid from the COD in the sewage stream, 2) separation of the acetic acid containing solution from the suspended solids, 3) recycling of the suspend solid fraction back into the fluidised bed bioreactor, 4) collection of hydrogen. The acetate containing effluent can be used to: 1) generate methane in a series of fluidised bed biofilm bioreactor-decanter-clarifier-gas separator assemblies or 2) generate hydrogen in a photofermenter containing selected cyanobacteria.

By manipulation of flow rate or dilution rate, temperature and pH, the fluidised bed bioreactor assemblies can be operated as chemostats that promote on a continuous basis the selection of extreme thermophilic anaerobic bacterial biofilm consortia that are pre-adapted to the chemical and physical nature of the primary sludge and which in addition have high reaction rates and capacities for the conversion of: 1) COD in the primary sewage sludge influent stream into hydrogen and acetic acid, 2) conversion of acetic acid into methane. The acetic acid containing effluent streams can be split into methane producing bioreactor and hydrogen producing photofermenters.

The method of the invention has the following features:
1. Sewage under normal circumstances is a dangerous substrate for biogas production and removal of pathogenic agents would be necessary before it can be safely used in a bioreactor process plant. Heat treatment of the primary sludge will remove most of the pathogens.
2. Heat treatment of the primary sludge and maintenance of the sewage stream at temperatures not below 70°C also selectively enriches the bioreactors with extreme thermophilic anaerobic bacterial consortia that are beneficial and necessary for the conversion of the COD in sewage into biogas for electricity generation.
3. The bioreactor systems when operated as chemostats facilitate the continuous enrichment and maintenance of bacterial biofilm consortia that are suitable for the maximum generation of hydrogen or methane from the substrates in the sewage stream. This type of bloreactor operation represents an opportunity to control the enrichment of the sewage stream with a suitable consortium of beneficial bacteria. In this case, beneficial with regard to hydrogen and methane production from sewage.
4. Enrichment with beneficial bacteria can be engineered so that occurs on a continuous basis.
5. This will facilitate the formation and maintenance of mixed bacterial consortia that are favourable for enhanced hydrogen and methane production within the bioreactor, whatever its configuration may be.
6. Maintenance of such a favourable mixed bacterial consortium by operating the bacteria is a chemostat will also dampen shocks that arise from variability in substrate concentration and composition.
7. Because the biofilm bed grows continuously the bed expands and biofilm coated carrier particles are washed out over the top of the bioreactor vessel. The biofilm particles are collected by sedimentation or settling in the clarifier and recycled back into the bioreactor.
8. Biofilm carrier particles lost from the bioreactor through attrition are also replenished by supplying fresh carrier medium to the influent stream. Together expanded biofilm bed washout, recycling of bed washout material back into the bioreactor and supplementation of fresh carrier medium results in the continuous turnover of the bioreactor biofilm bed in the bioreactor vessel.
9. Continuous re-inoculation of the process stream by recycling from the clarifier with a mixed community of bacteria will make the process operation robust and reproducible.
10. Enrichment of the process stream with a mixed community of beneficial bacteria will be achieved by recycling of the insoluble suspended bioreactor effluents from the clarifier back into the sewage influent stream.
11. This enrichment of the process stream will also be achieved by continuous re-inoculation of the bioreactor influent stream with bacterial biofilm coated particles that are collected from the bioreactor overflow into the clarifier.
12. Both facultative and obligately anaerobic bacteria will be produced by these bioreactors. The facultative anaerobic bacteria will also remove oxygen and help maintain anaerobiosis.
13. The recycle-chemostat method of bioreactor operations results in the continuous enrichment of wastewater feed streams with obligate and facultative anaerobic thermophilic bacteria selected for their high capacity for hydrogen or methane production and which in addition have also been pre-adapted to the chemical and physical properties of the wastewater stream.
14. Acetic acid is a major product of the hydrogen producing bioreactors. Acetic acid can be used as substrate for hydrogen production in a photobioreactor.

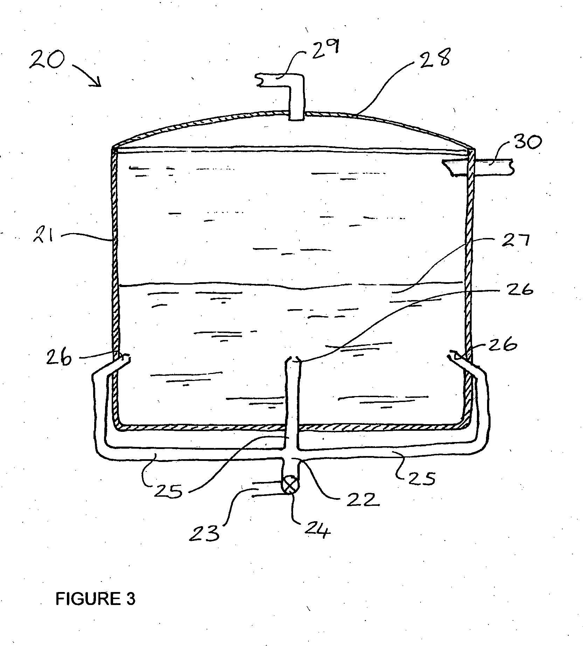

Referring now to Figure 3, a fluidised bed biofilm reactor (20) according to the invention comprises a reactor vessel (21) having an inlet manifold (22) which receives wastewater form a wastewater heater (not shown) via a conduit (23). Wastewater is heated in the heater to a temperature of 95°C for a period of between 10 and 20 minutes after which the wastewater is allowed to cool to a temperature of between 70 and 80°C before being pumped into the reactor (21) by a pump (24) in the conduit (23).

In the embodiment illustrated the manifold (22) splits into five branches (25) of which three are shown. Each branch (25) terminates in a nozzle (26).which projects into the reactor vessel (21). In this embodiment there are five nozzles (26), one central nozzle which feeds into the bottom of the reactor vessel (21) and four circumferentially spaced nozzles mounted in the sides of the reactor vessel (21). All of the nozzles (26) are orientated operatively upwardly and, in use, direct the wastewater inflow towards a biofilm (27) where thermophilic anaerobic bacteria convert carbohydrates, fats and proteins in the wastewater into biogas which collects in the domed top (28) of the reactor vessel (21) and is led off *via* a conduit (29). Treated wastewater is led off *via* a conduit (30) to be returned to the environment.

The temperature in the reactor (21) is between 70 and 80°C and the wastewater in the reactor has a retention time which allows the temperature to be maintained.

## Claims

1. A process for the production of hydrogen and methane from sewage wastewater streams thus, at least partly, rendering said wastewater safe for use in a bioreactor process plant and rendering the wastewater suitable for the production of biological gasses, said method comprising receiving a primary untreated wastewater stream (16), heating said untreated wastewater stream to a first predetermined temperature of 95°C for between 10 and 20 minutes to, in use, inactivate pathogenic micro-organisms in the wastewater stream, allowing the heated wastewater to cool to a second predetermined temperature which, in use, facilitates growth, in the wastewater stream, of the anaerobic thermophilic bacteria, maintaining the second predetermined temperature of the wastewater stream while passing said stream through a fluidised bed biofilm reactor (17, 21) within which are desired anaerobic thermophilic bacteria, decanting, clarifying and separating fermentation products produced in the reactor from the wastewater outflow (18) from the bioreactor and returning the treated wastewater to the environment, in which process hydrogen and/or methane produced by the anaerobic thermophilic bacteria is used to provide at least part of the energy required to heat the untreated wastewater stream to its first predetermined temperature and/or to maintain the second predetermined temperature of the wastewater stream.

2. A process for the production of hydrogen and methane from sewage wastewater streams as claimed in claim 1 in which the second predetermined temperature is not less than 70°C, preferably between 70°C and 80°C.

3. A process for the production of hydrogen and methane from sewage wastewater streams as claimed in any one of the preceding claims in which the wastewater stream (16) is continuously enriched with appropriate anaerobic thermophilic bacteria after it has cooled to its second predetermined temperature, wherein preferably the wastewater stream is enriched by inoculation.

4. A process for the production of hydrogen and methane from sewage wastewater streams as claimed in claim 3 in which the appropriate anaerobic thermophilic bacteria have a high capacity for hydrogen and/or methane production.

## Patentansprüche

1. Verfahren zur Herstellung von Wasserstoff und Methan aus Abwasserströmen, demzufolge, zumindest teilweise, dieses Abwasser sicher zum Gebrauch in einer Bioreaktor-Aufbereitungsanlage gemacht wird und das Abwasser für die Produktion von biologischen Gasen geeignet gemacht wird,
wobei dieses Verfahren die Aufnahme eines anfänglich unbehandelten Abwasserstroms (16), das Erhitzen dieses unbehandelten Abwasserstroms auf eine erste vorbestimmte Temperatur von 95 °C für zwischen 10 und 20 Minuten, um im Betrieb krankheitserregende Mikroorganismen in dem Abwasserstrom zu inaktivieren, das Abkühlenlassen des erhitzten Abwassers auf eine zweite vorbestimmte Temperatur, die im Betrieb ein Wachstum von anaeroben, thermophilen Bakterien im Abwasserstrom ermöglicht, das Aufrechterhalten der zweiten vorbestimmten Temperatur des Abwasserstroms während dieser Strom durch einen Wirbelschichtreaktor mit Biofilm (17, 21) geleitet wird, in welchem sich die gewünschten anaeroben, thermophilen Bakterien befinden, das Dekantieren, das Klären und das Abtrennen von Gärungsprodukten, die in dem Reaktor vom Ablauf des Abwassers (18) aus dem Bioreaktor produziert werden, und die Rückgabe des behandelten Abwassers in die Umwelt umfasst,
wobei in dem Verfahren Wasserstoff und/oder Methan, das von den anaeroben, thermophilen Bakterien hergestellt wird, genutzt wird, um zumindest einen Teil der Energie, die zum Erhitzen des unbehandelten Abwassers auf seine erste vorbestimmte Temperatur und/oder zum Aufrechterhalten der zweiten vorbestimmten Temperatur des Abwasserstroms benötigt wird; bereitzustellen.

2. Verfahren zur Herstellung von Wasserstoff und Methan aus Abwasserströmen wie in Anspruch 1 beansprucht, in dem die zweite vorbestimmte Temperatur nicht geringer als 70 °C, vorzugsweise zwischen 70 °C und 80 °C, ist.

3. Verfahren zur Herstellung von Wasserstoff und Methan aus Abwasserströmen wie in irgendeinem der vorstehenden Ansprüche beansprucht, in dem der Abwasserstrom (16), nachdem er auf seine zweite vorbestimmte Temperatur abgekühlt ist, kontinuierlich mit geeigneten anaeroben, thermophilen Bakterien angereichert wird, wobei der Abwasserstrom vorzugsweise durch Inokulation angereichert wird.

4. Verfahren zur Herstellung von Wasserstoff und Methan aus Abwasserströmen wie in Anspruch 3 beansprucht, wobei die geeigneten anaeroben, thermophilen Bakterien eine hohe Kapazität zur Wasserstoff- und/oder Methanproduktion haben.

## Revendications

1. Processus destiné à la production d'hydrogène et de méthane à partir de flux d'eaux usées d'égouts, rendant ainsi, au moins partiellement, lesdites eaux usées sans danger pour une utilisation dans une installation de processus de bioréacteur et rendant les eaux usées adaptées à la production de gaz biologiques, ledit procédé comprenant le fait de recevoir un flux primaire d'eaux usées non traitées (16), de chauffer ledit flux d'eaux usées non traitées jusqu'à une première température prédéterminée de 95°C pendant une durée comprise entre 10 et 20 minutes pour, en cours d'utilisation, inactiver les micro-organismes pathogènes dans le flux d'eaux usées, de permettre le refroidissement des eaux usées chauffées jusqu'à une deuxième température prédéterminée qui, en cours d'utilisation, facilite la croissance des bactéries thermophiles anaérobies dans le flux d'eaux usées, de maintenir la deuxième température prédéterminée du flux d'eaux usées lors du passage dudit flux à travers un réacteur à film biologique à lit fluidisé (17, 21) à l'intérieur duquel se trouvent les bactéries thermophiles anaérobies désirées, de faire décanter, de clarifier et de séparer des produits de fermentation produits dans le réacteur du flux d'évacuation d'eaux usées (18) à partir du bioréacteur et de renvoyer les eaux usées traitées dans l'environnement, où l'hydrogène et/ou le méthane du processus produit(s) par les bactéries thermophiles anaérobies est/sont utilisé(s) pour fournir au moins une partie de l'énergie nécessaire pour chauffer le flux d'eaux usées non traitées jusqu'à sa première température prédéterminée et/ou pour maintenir la deuxième température prédéterminée du flux d'eaux usées.

2. Processus destiné à la production d'hydrogène et de méthane à partir de flux d'eaux usées d'égout tel que revendiqué dans la revendication 1, dans lequel la deuxième température prédéterminée n'est pas inférieure à 70°C, de préférence elle est comprise entre 70°C et 80°C.

3. Processus destiné à la production d'hydrogène et de méthane à partir de flux d'eaux usées d'égout tel que revendiqué dans l'une quelconque des revendications précédentes, dans lequel le flux d'eaux usées (16) est enrichi en continu avec des bactéries thermophiles anaérobies appropriées après son refroidissement jusqu'à sa deuxième température prédéterminée, dans lequel, de préférence, le flux d'eaux usées est enrichi par inoculation.

4. Processus destiné à la production d'hydrogène et de méthane à partir de flux d'eaux usées d'égout tel que revendiqué dans la revendication 3, dans lequel les bactéries thermophiles anaérobies appropriées ont une grande capacité de production d'hydrogène et/ou de méthane.
